# EUROPÄISCHE PATENTSCHRIFT

(11) **EP 0 555 639 B1**
(45) Veröffentlichungstag und Bekanntmachung des Hinweises auf die Patenterteilung: **05.04.1995**
(21) Anmeldenummer: 93100364.4
(22) Anmeldetag: 13.01.1993
(51) Int. Cl.: D02G 1/02, G01N 33/36

(54) **Verfahren zur Regelung der Fadenzugkraft des laufenden Fadens in einer Falschzwirntexturiermaschine**
Method for controlling the yarn tension of travelling yarns on a falsetwist texturing machine
Procédé pour le réglage de la force de tension des fils en mouvement sur une machine à texturer par fausse torsion

(30) Priorität: 10.02.1992 DE 4203788
(43) Veröffentlichungstag der Anmeldung: 18.08.1993
(73) Patentinhaber: BARMAG AG, D-42862 Remscheid (DE)
(72) Erfinder: Neumann, Bernd, W-5608 Radevormwald (DE); Lorenz, Hellmut, W-5630 Remscheid 1 (DE); Wessolowski, Bernd, W-5630 Remscheid (DE)
(74) Vertreter: Pfingsten, Dieter, Dipl.-Ing.

(56) Entgegenhaltungen:
- WO-A-92/11535
- US-A- 4 896 407
- US-A- 4 961 308

## Beschreibung

Die Erfindung betrifft ein Verfahren zur Regelung der Fadenzugkraft des laufenden Fadens in einer Falschzwirntexturiermaschine nach dem Oberbegriff des Anspruches 1.

Ein derartiges Verfahren ist bekannt durch die WO-A-92/11535 vom 14.12.1991, veröffentlicht am 9.7.1992.

Die Anwendung des bekannten und älteren Verfahrens ist problemlos während des Normalbetriebes, wenn der Faden seine Sollgeschwindigkeit und Solltemperatur hat.

Die Aufgabe der Erfindung besteht darin, die Verfahren den außerhalb des Normalbetriebes liegenden Ausnahme-Betriebssituationen anzupassen. Als solche Ausnahme-Situationen sind insbesondere anzusehen das Fadenanlegen sowie die Betriebsstörung durch kurzfristigen Elektrizitätsausfall.

Als Betriebsphase des Fadenanlegens ist das Einfädeln des Fadens in seine Bearbeitungsposition, d. h. in die Lieferwerke, den Friktionsfalschdraller, die Heizschiene und Kühlschiene, darüber hinaus aber auch die daran anschließende Betriebsphase zu sehen, in der der Faden seinen Sollbetriebsrahmen noch nicht erreicht hat.

Kurzzeitige Störungen der Elektrizitätsversorgung sind verhältnismäßig häufig. Sie sollten nicht zu einem Fadenbruch führen, da sonst sämtliche Fäden der Texturiermaschine neu angelegt werden müßten. Das ist insbesondere dann störend, wenn die Fäden durch einen längs der Maschinenfront beweglichen Automaten (Doffer) neu angelegt werden müßten.

Die Lösung der solcher Art gestellten Aufgabe ergibt sich aus Anspruch 1.

Der besondere Vorteil der Lösung ist darin zu sehen, daß die Texturiermaschine besser einem automatischen Betrieb, d.h. deren Bedienung durch einen Doffer angepaßt wird.

Die Reibkraft kann dabei zwischen 0 und ihrem Sollwert vorgegeben werden.

Die Erfindung hat insbesondere erkannt, daß sehr häufig Störungen in der Elektrizitätsversorgung vorkommen, die von so kurzer Zeitdauer sind, daß zwar noch kein Fadenbruch eintritt, daß jedoch spürbare Qualitätseinbrüche mit Fehlern im texturierten Garn zu befürchten sind.

Diese Qualitätseinbrüche beruhen auf einer Regelkreisauslegung, die insbesondere in Betriebspunktnähe stabil sein soll, so daß sich Überschwingen des Regelkreises beim plötzlichen Stromausfall nur mit aufwendiger Anpassung der Regelparameter vermeiden läßt.

Diesen Aufwand kann die Erfindung jedoch vermeiden.

Der Vorzug der Lösung nach Anspruch 2 besteht darin, daß zum einen eine Überlastung des Fadens vermieden wird, der Faden jedoch zum anderen durch Zwirn eine erhöhte Festigkeit in der kritischen Phase außerhalb des Normalbetriebes (Fadenanlegen, Betriebsstörung) erhält.

Dabei macht sich diese Weiterbildung die folgende Erkenntnis zunutze:
Insbesondere in der kritischen Anlegphase kann infolge des bereits erfolgenden Drallaufbaus mit einem Abbau der Zugkraftspitzen gerechnet werden, so daß sich dieses Verfahren insbesondere zum Anlegen des Fadens bei hohen und höchsten Fadenlaufgeschwindigkeiten eignet. Dabei erfolgt der Drallaufbau durch die quer zum Faden liegenden Komponenten der Reibkraft allmählich, während die Komponenten der Reibkraft in Fadenförderrichtung bereits wirksam werden und den Faden aus dem Stillstand auf eine Geschwindigkeit zwischen NULL und Betriebsgeschwindigkeit beschleunigen.

Damit konnte eine drastische Reduzierung der Fehlversuche beim Fadenanlegen erreicht werden.

Die Ausführung nach Anspruch 2 bietet darüber hinaus den Vorteil, daß ein allmählicher Übergang der Fadenbelastung zwischen der Betriebsphase des Fadenanlegens und dem normalen Betriebszustand erreicht wird, wodurch die möglichen Zugkraftspitzen lediglich in kleinen Stufen auftreten können.

Die Übergangszeit beim und nach dem Fadenanlegen, während welcher die Fadenzugkraft-Regelung nicht in Betrieb ist, kann konstant vorgegeben werden.

Anspruch 4 bietet jedoch die Möglichkeit, unzulässige Fadenbelastung durch Anpassung an den jeweiligen Betriebszustand des Fadens zu vermeiden.

Dies wird dadurch erreicht, daß die Umschaltung vom ungeregelten Betrieb in der Anlegphase in den geregelten Zustand zu einem festen Zeitpunkt erfolgt, der durch das Erreichen des Zugkraft-Sollbereichs vorgegeben ist. Der Zugkraft-Sollbereich kann so vorgegeben sein, daß die Umschaltung zum frühest möglichen Zeitpunkt erfolgt.

In diesem Fall bietet es sich an, diesen Zeitpunkt so zu bestimmen, daß er am Ende des Zeitraums liegt, in welchem der allmähliche Drallaufbau erfolgt. Demgemäß kann die Umschaltung erfolgen, sobald der Drallzustand stationär ist.

Die älteren bzw. bekannten Verfahren haben die Besonderheit, daß auch eine Qualitätsüberwachung des laufenden Fadens möglich ist.
Die Fadenzugkraftregelung sowie die Qualitätssicherung, die funktionell eng und sachgemäß ineinandergreifen, werden mit dem von der Auswerteinrichtung gewonnenen Ausgangssignal aktiviert.

Demselben Ziel dient auch die Weiterbildung nach Anspruch 5.

Anspruch 6 stellt eine Weiterbildung von Anspruch 4 dar, mit dem Vorteil, daß insbesondere die Fadenzugkraftschwankungen, die im stationären Fadenzustand sehr klein sein können, erfasst werden und für die Ingangsetzung der Fadenzugkraftregelung ausgewertet werden.

Anspruch 7 bietet eine Möglichkeit, absolute Meßergebnisse für das Auswertsignal heranzuziehen, die insbesondere bei hohen Differenzen zwischen Meßsignal und Mittelwert, also bei hohen Fadenzugkraftspitzen, aussagekräftiger sein können als die relative Standardabweichung.

Anspruch 8 betrifft eine Weiterbildung der Erfindung, der die Erkenntnis zugrunde liegt, daß zwischen verschiedenen Einflußgrößen des Regelkreises und der Reibkraft des Friktionsfalschdrallers funktionelle Zusammenhänge bestehen, die entsprechend den Betriebsbedingungen, z.B. Fadengeschwindigkeit, Verteilung der Reibkraftkomponenten in Fadenrichtung und quer zur Fadenrichtung, Fadenbeschaffenheit, Fadenfestigkeit, Zwirnverhalten, für optimale Ergebnisse vorgewählt werden können.

Es kommt also darauf an, daß jeweils ein bestimmter Zusammenhang zwischen der jeweiligen Stellgröße und der Reibkraft besteht und daß für verschiedene Stellgrößen verschiedene mathematische Funktionen des Reibkraftverlaufs vorliegen können.

Anspruch 9 betrifft eine Weiterbildung mit dem Vorteil einer individuellen Reibkraftvorgabe für Betriebsphasen unterschiedlicher Fadenanforderungen.

Anspruch 10 deckt die Mehrzahl praktisch auftretender Ausnahme-Betriebssituationen ab, wobei die Ansprüche 11 und 12 die Reibkraft über die jeweils zwischen den Reibflächen des Friktionsfalschdrallers wirkenden Normalkräfte berücksichtigen, insbesondere nach Anspruch 11 die Möglichkeit eröffnen, die Komponenten der Reibkraft quer zur und in Fadenlaufrichtung unabhängig oder abhängig voneinander vorzugeben.

Die Weiterbildung nach Anspruch 13 verdient besondere Aufmerksamkeit, da die Drehzahl des Friktionsfalschdrallers besonders einfach zu erfassen, zwischenzuspeichern und wieder abzurufen ist. Der mathematische Zusammenhang zwischen der Drehzahl und der Reibkraft ist darüberhinaus stets der gleiche, so daß über lange Zeit gesehen reproduzierbare bzw. vorhersagbare Produktionsergebnisse zu erwarten sind.

Es soll ausdrücklich gesagt sein, daß diese Merkmale insbesondere in Verbindung mit den Merkmalen der Ansprüche 11 bzw. 12 zur Erfindung gehören.

Die Merkmale des Anspruchs 14 stellen einerseits die Regelbarkeit bei Stromunterbrechung sicher, vermeiden andererseits ungünstiges Schwingungsverhalten des Regelkreises, z.B. Überschwingen.

Anspruch 15 gibt möglichst genau den letzten Parameterwert der Stellgröße wieder, wodurch die Stellgröße in nächstmöglicher Nähe zum Betriebspunkt bereitgestellt wird, was insbesondere für sehr kurzzeitige Stromunterbrechungen vorteilhaft ist.

In allen Fällen mit vorgegebener Stellgröße entsprechend den Ansprüchen 8 bis 15 liegen übliche Werte der Stellgröße zwischen NULL und dem Betriebs-Sollwert, so daß von einem stabilen Erreichen des Betriebspunktes auszugehen ist.

Im folgenden wird ein Auswertungsbeispiel der Erfindung anhand der Zeichnung beschrieben.

Es zeigen:
- Fig. 1: eine Bearbeitungsstelle einer Falschzwirnkräuselmaschine in schematischer Darstellung;
- Fig. 2: ein Diagramm der Fadenzugkraft in der Anleg-Phase

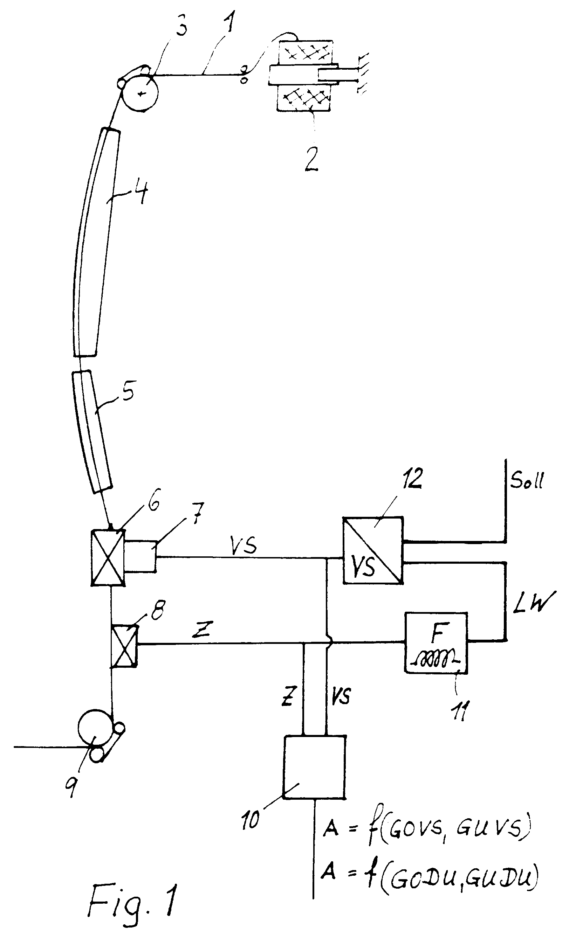

Die Figur 1 zeigt eine schematische Darstellung einer Bearbeitungsstelle einer Falschzwirnkräuselmaschine. Der synthetische Faden 1 wird durch das Eingangslieferwerk 3 von der Vorlagespule 2 abgezogen. Die Texturierzone wird zwischen dem Eingangslieferwerk 3 und dem Abzugslieferwerk 9 gebildet. Sie umfaßt vor allem eine Heizschiene 4, eine Kühlschiene 5 und den Friktionsfalschdraller 6. Der Friktionsfalschdraller weist endlos bewegte Oberflächen auf, die quer zur Fadenachse bewegt sind und an denen der Faden anliegt. Diese Oberflächen erteilen dem Faden in Richtung Eingangslieferwerk eine Zwirnung, die sich in Richtung des Ausgangslieferwerks 9 wieder auflöst.
Darüberhinaus wird dem Faden mittels der Oberflächen auch eine in Fadenrichtung wirksame Förderkomponente der Reibkraft aufge-zwungen.

Zwischen dem Friktionsfalschdraller 6 und dem Ausgangslieferwerk 9 ist ein Fadenzugkraftmeßinstrument 8 angeordnet, durch welches die Fadenzugkraft, auch "Fadenspannung" genannt, gemessen und als Ausgangssignal weitergegeben wird. Es sei bemerkt, daß hinter dem Ausgangslieferwerk 9 eine Aufwicklung oder noch eine Zwischenbehandlung durch Erwärmung folgt.

Das Ausgangssignal des Fadenzugkraftmessers 8, welches die gemessene Fadenzugkraft repräsentiert, wird über einen Filter 11 in einen Langzeitwert LW umgeformt. Der Langzeitwert LW wird gemeinsam mit einem Sollwert einer Regeleinrichtung 12 zugeführt. In der Regeleinrichtung 12 werden der Sollwert und der Langzeitwert miteinander verglichen und in eine Verstellgröße VS umgeformt. Durch die Verstellgröße VS wird ein Stellglied 7 verstellt, durch welches die Drallübertragung des Friktionsfalschdrallers 6 auf den Faden 1 gesteuert wird.

Dabei wird auch die in Fadenrichtung liegende Förderkomponente der Reibkraft beeinflußt.

Das Ausgangssignal des Fadenzugkraftmessers 8 wird ebenso wie das Verstellsignal VS einer Auswerteinrichtung 10 aufgegeben. In der Auswerteinrichtung 10 repräsentiert das Verstellsignal VS den Mittelwert der Fadenzugkraft. Die Auswerteinrichtung 10 liefert eine Auswertung des aktuellen Ausgangssignals, welches die aktuell gemessene Fadenzugkraft repräsentiert, entsprechend den Grundsätzen, die in der EP 207 471 A1 beschrieben sind.

Das bedeutet: In der Auswerteinrichtung 10 ist ein oberer Grenzwert und ein unterer Grenzwert für das Verstellsignal VS eingespeichert (GOVS, GUVS). Wenn das Verstellsignal VS einen dieser Grenzwerte überschreitet, erfolgt ein Alarmsignal, ggf. eine Fehlermeldung. Ferner wird in der Auswerteinrichtung 10 der Differenzwert DU zwischen dem aktuellen Ausgangssignal und dem Verstellsignal VS - nachdem beide zuvor in kompatible, vergleichbare Größen gewandelt worden sind - gebildet. Schließlich ist in der Auswerteinrichtung 10 der obere Grenzwert und der untere Grenzwert dieses Differenzsignales DU (GODU, GUDU) gespeichert, und es erfolgt ein Alarmsignal A, wenn das Differenzsignal DU zwischen dem Verstellsignal und dem aktuell gemessenen Ausgangssignal einen der Grenzwerte GODU, GUDU überschreitet.

Schließlich ist es auch möglich und in der Anlegphase vorteilhaft, entsprechend EP-A-0 495 446 die Standardabweichung der Fadenzugkraft zu ermitteln. Hierzu wird die Fadenzugkraft gemessen. Der Meßwert wird durch einen Filter in einen Mittelwert der Fadenzugkraft umgeformt. Das Meßsignal und sein laufend gebildeter Mittelwert werden subtrahiert und die Differenz quadriert. Aus dieser quadrierten Differenz wird durch Wurzelbildung die Standardabweichung S gebildet.

Die Verstelleinrichtung 7 kann z. B. der Antriebsmotor des Friktionsfalschdrallers sein. In diesem Falle erfolgt in der Einrichtung 12 eine Umwandlung des Differenzsignals aus dem Langzeitwert und dem Sollwert in eine Stellgröße, z.B. in eine Frequenz, die die Drehzahl des als Synchronmotor oder als Asynchronmotor ausgelegten Antriebsmotors 7 des Friktionsfalschdrallers bestimmt.

Wenn der Friktionsfalschdraller aus drei parallelen Achsen besteht, die in den Eckpunkten eines gleichseitigen Dreiecks angeordnet sind und auf welchen Scheiben aufgespannt sind, die sich im Zentrum des Dreiecks überlappen, so kann durch die Verstelleinrichtung 7 alternativ oder zusätzlich der Achsabstand verstellt werden, indem z.B. die Achsen auf jeweils einem Exzenter gelagert und die Exzenter in Abhängigkeit von dem Verstellsignal VS entsprechend verdreht werden (vgl. DE-AS 21 30 550).

Wenn der Friktionsfalschdraller aus zwei Scheiben besteht, die den Faden zwischen sich einklemmen und von denen eine elastische Scheibe durch eine Andrückeinrichtung gegen die andere Scheibe gedrückt wird (vgl. z.B. EP 22 743 A1), so kann durch das Verstellsignal VS die Anpreßkraft verstellt werden. Hierzu wird auch auf die DE 33 06 594 A1 verwiesen.

Wenn der Friktionsfalschdraller aus zwei Riemen besteht, die endlos quer zur Fadenachse umlaufen und den Faden zwischen sich einklemmen (z.B. US 4,248,038), wobei die Riemenlagerungen mit vorgebbarer Kraft relativ zueinander verstellt werden, so kann durch das Verstellsignal VS diese Kraft verstellt werden.

Zumindest für die obengenannten Friktionsfalschdraller unterschiedlicher Bauformen, soll ausdrücklich darauf hingewiesen werden, daß die Reibkraft durch eine vorgegebene Drehzahl vorgegeben werden kann, da bei Friktionsfalschdrallern ein Zusammenhang zwischen Reibkraft und Drehzahl besteht.

Dabei kann die Drehzahl entweder als alleinige Stellgröße oder als zusätzliche Stellgröße zu den bereits genannten Maßnahmen dienen.

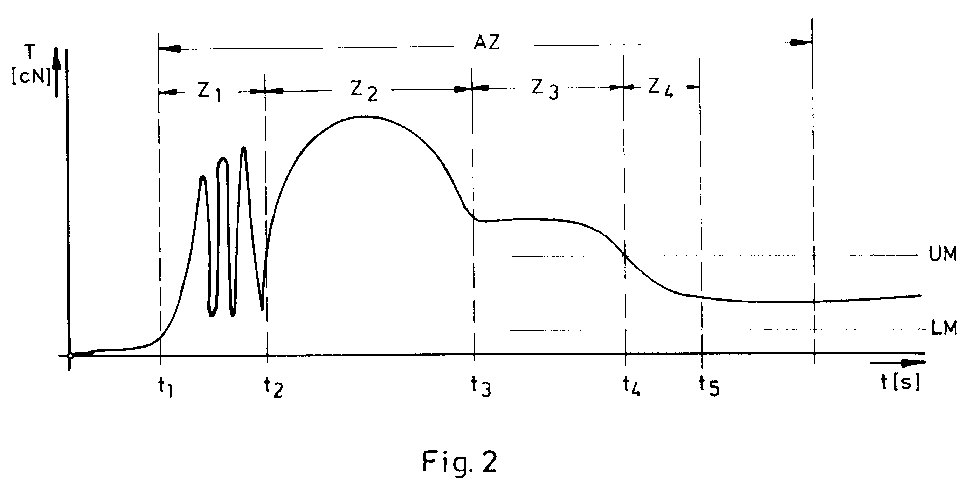

Anhand des Diagrammes nach Fig. 2 wird das Fadenanlegen beschrieben.

Im folgenden wird der Anlegvorgang in die Zeiten Z1 bis Z4 unterteilt.

In der Zeit Z1 wird der Faden von der Vorlagespule 2 mittels einer Anlegpistole von der Vorlagespule abgezogen und in das Lieferwerk 9, den Friktionsfalschdraller 6, den Fadenzugkraftmesser 8 eingeführt. Dabei ist die Verstelleinrichung 7 des Friktionsfalschdrallers 6 jedoch nicht im Regelbetrieb sondern auf einen konstanten Wert eingestellt, der z.B. gleich dem oder niedriger als als der Betriebs-Sollwert ist.

Ist dieser Wert niedriger als der Betriebs-Sollwert, erfolgt daher bereits ein Aufbau der Zwirnung mit einer Drallsäule geringer Dralldichte.

Der Fadenzugkraftmesser 8 zeigt bereits einen Ausschlag, auch wenn das Lieferwerk 3 noch nicht aktiviert ist. Der Fadenlauf ist jedoch sehr instabil. Daher sind die Fadenzugkraftschwankungen, ermittelt in Form des CV-Wertes, sehr hoch.

Anlegzeit Z2: Nunmehr wird der Faden, der noch immer in die Saugpistole läuft, an die Heizschiene 4 und die Kühlschiene 5 angelegt. Das Lieferwerk 3 wird geschlossen, so daß der Faden nunmehr durch die Einspannung zwischen den Lieferwerken 3 und 9 und ihre Geschwindigkeitsdifferenz sowie durch den zunehmenden Aufbau des Dralles einer erhöhten Fadenzugkraft ausgesetzt wird. Der Fadenlauf wird allerdings stabiler, so daß die Standardabweichung geringer wird.

Anlegzeit Z3: der Fadenlauf hat sich stabilisiert. Die Standardabweichung unterschreitet einen vorbestimmten Grenzwert. Die Anlegzeit Z3 ist daher eine Testzeit. In dieser Testzeit wird erfasst, ob der Grenzwert der Standardabweichung noch überschritten wird. Wenn die Standardabweichung während der Testzeit den Grenzwert nicht überschreitet, wird nach Ablauf der Testzeit die Fadenzugkraftregelung eingeschaltet, indem der Fadenzugkraftmesser 8 und die Verstelleinrichtung 7 des Friktionsfalschdrallers 6 in einen Regelkreis eingeschlossen werden. Die Verstelleinrichtung, durch welche die Reibkraft des Friktionsfalschdrallers 6 vorgegeben wird, wird so verstellt, daß der Fadenzugkraftmesser 8 eine im wesentlichen konstante Fadenzugkraft zeigt.

Wenn während der Testzeit doch noch Störungen auftreten, wird die Testzeit Z3 nach ihrem Ablauf neu gestartet.

Anlegzeit Z4: Nach erfolgreichem Abschluß der Testzeit für die Standardabweichung arbeitet die Fadenzugkraftregelung mit einem vorgegebenem Sollwert der Fadenzugkraft. Während der Anlegzeit Z4 stellt sich die Verstelleinrichtung 7 so ein, daß die Fadenzugkraft ihren Sollwert erreicht.

Nach Ablauf der zuvor beschriebenen Testzeit Z3 wird die Qualitätsüberwachung in Gang gesetzt. Es erfolgt nunmehr eine Überwachung der Fadenzugkraft mit den zuvor beschriebenen Maßnahmen. Dabei wird also überwacht, ob die Fadenzugkraft oder daraus abgeleitete Auswertsignale und/oder das der Verstelleinrichtung 7 vorgegebene Verstellsignal vorgegebene Toleranzbereiche verläßt. Diese Ereignisse können allein oder gesamt zur Qualitätsbestimmung der erzeugten Spule registriert werden.

Es besteht nun z.B. durch falsche Maschineneinstellung oder Fehler von Maschinenteilen die Möglichkeit, daß während der Testzeit Z3 der vorgegebene Sollwert der Fadenzugkraft und/oder der Sollwert der Verstellkraft nicht erreicht wird. In diesem Fall wird nach Ablauf einer Gesamt-Anlegzeit, die größer ist als die Summe der vorbeschriebenen Zeiten Z1 - Z4, die Qualitätsüberwachung in jedem Fall eingeschaltet. Dadurch wird gewährleistet, daß bei falscher Maschineneinstellung oder Fehlern der Maschine, Qualitätsfehler der Spule erkannt und beseitigt werden können.

Auf jeden Fall kann mit den durch die Erfindung bereitgestellten Mitteln bei Qualitätsabweichungen durch AusnahmeBetriebssituationen sofort reagiert werden um die Sollqualität zu erreichen. Andernfalls besteht immer die Möglichkeit, einen Fadenschnitt anzubringen, wenn die Qualitätsfehler nicht ausgeregelt werden können.

## Patentansprüche

1. Verfahren zur Regelung der Fadenzugkraft des laufenden Fadens (1) in einer Falschzwirntexturiermaschine,
mit einer Vorlagespule (2), von der der Faden (1) abgezogen wird, mit einem ersten Lieferwerk (3),
einer Heizschiene (4),
einer Kühlschiene (5),
einem Friktionsfalschdraller (6)
einem Reibkraft-Stellglied (7) und
mit einem Fadenzugkraftmeßgerät (8), durch dessen Ausgangssignal während des Normalbetriebs die Reibkraft des Friktionsfalschdrallers (6) mittels des Reibkraftstellgliedes (7) zur Regelung der Fadenzugkraft verstellbar ist,
dadurch gekennzeichnet, daß
außerhalb des Normalbetriebes, insbesondere beim Auslauf der Maschine bei Stromunterbrechung und/oder beim Anlegen des Fadens (1) die Regelung der Fadenzugkraft ausgeschaltet und eine ungeregelte Reibkraft vorgegeben wird.

2. Verfahren nach Anspruch 1,
dadurch gekennzeichnet, daß
die Reibkraft auf einen Wert größer Null jedoch niedriger als der Betriebswert eingestellt wird und danach vorzugsweise stetig oder in Stufen erhöht wird.

3. Verfahren nach Anspruch 1,
dadurch gekennzeichnet, daß
zum Fadenanlegen nach einer konstant vorgegebenen Anlaufzeit die Fadenzugkraftregelung eingeschaltet wird.

4. Verfahren nach Anspruch 1,
dadurch gekennzeichnet, daß
die Fadenzugkraftregelung in Abhängigkeit von der Messung der Fadenzugkraft und einem daraus gewonnenen Auswert-Signal bei Erreichen eines vorgegebenen Zugkraft-Sollbereiches eingeschaltet wird.

5. Verfahren nach einem der Ansprüche 3 bis 5,
dadurch gekennzeichnet, daß
eine Qualitätsüberwachung des laufenden Fadens (1) durch Auswertung der Fadenzugkraft erfolgt, wenn die Fadenzugkraft einen vorgegebenen Soll-Bereich erreicht hat und vorzugsweise, wenn danach eine vorgegebene Regel-Testzeit Z3 abgelaufen ist.

6. Verfahren nach Anspruch 5,
dadurch gekennzeichnet, daß
das Auswertsignal die laufende relative Standardabweichung (Variationskoeffizient (cv)) ist, welche aus dem laufenden Meßwert der Fadenzugkraft und dem daraus laufend gebildeten Mittelwert gebildet wird.

7. Verfahren nach Anspruch 5,
dadurch gekennzeichnet, daß
das Auswertsignal die Standardabweichung S ist, welche aus der Wurzel der quadrierten Differenz zwischen dem laufenden Meßwert der Fadenzugkraft und deren Mittelwert gebildet wird.

8. Verfahren nach einem der Ansprüche 1 bis 8,
dadurch gekennzeichet, daß
die ungeregelte Reibkraft durch eine Stellgröße aus dem Regelkreis der Fadenzugkraft vorgegeben wird.

9. Verfahren nach Anspruch 9,
dadurch gkennzeichnet,daß
die Stellgröße jeweils in Abhängigkeit von der jeweiligen Ausnahme-Betriebssituation unterschiedlich vorwählbar ist.

10. Verfahren nach Anspruch 10,
dadurch gekennzeichnet, daß
die Stellgröße im Falle der Stromunterbrechung unterschiedlich zur Stellgröße im Falle des Fadenanlegens wählbar ist.

11. Verfahren nach einem der Ansprüche 9 bis 11,
dadurch gekennzeichnet, daß
eine Stellgröße vorgegeben wird, die im wesentlichen die auf den Faden (1) wirkenden Komponenten der Reibkraft bestimmt.

12. Verfahren nach einem der Ansprüche 9 bis 11,
dadurch gekennzeichnet, daß
eine Stellgröße vorgegeben wird, die im wesentlichen die Anpreßkraft des Friktionsfalschdrallers (6) bestimmt.

13. Verfahren nach einem der Ansprüche 9 bis 13,
dadurch gekennzeichnet, daß
eine drehzahlabhängige Stellgröße vorgegeben wird, deren Vorgabewert unterhalb des Sollwerts bei Betriebsdrehzahl liegt.

14. Verfahren nach Anspruch 9 bis 14,
dadurch gekennzeichnet, daß
bei Stromunterbrechung der Regelkreis von einem elektrischen Notstromnetz gepuffert wird, und daß
als ungeregelte Stellgröße ein vorbestimmter Parameterwert in der Nähe des Betriebspunktes vorgegeben wird.

15. Verfahren nach Anspruch 15,
dadurch gekennzeichnet, daß
der Parameterwert der Stellgröße im Normalbetrieb fortlaufend ermittelt und gespeichert wird, und daß
bei Stromunterbrechung der zuletzt gespeicherte Parameterwert die ungeregelte Stellgröße repräsentiert.

## Claims

1. Method of controlling the yarn tension of the continuous yarn (1) in a false twist texturing machine, having a feed bobbin (2) from which the yarn (1) is removed, having a first delivery mechanism (3), a heating rail (4), a cooling rail (5), a friction false-twisting device (6), a friction force final controlling element (7) and a yarn tension measuring instrument (8), by means of whose output signal during normal operation the friction force of the friction false-twisting device (6) is adjustable with the aid of the friction force final controlling element (7) to control the yarn tension,
characterized in that
outside of normal operation, in particular upon coasting of the machine in the event of a power cut and/or upon piecing of the yarn (1), the yarn tension control system is switched off and an uncontrolled friction force is preselected.

2. Method according to claim 1,
characterized in that
the friction force is set at a value greater than zero but lower than the operating value and is then increased preferably continuously or in steps.

3. Method according to claim 1,
characterized in that
for yarn piecing, after a constant preselected start-up time the yarn tension control system is switched on.

4. Method according to claim 1,
characterized in that
the yarn tension control system is switched on in dependence upon the measurement of the yarn tension and an evaluation signal obtained therefrom when a preselected tension setpoint range is reached.

5. Method according to one of claims 3 to 5,
characterized in that
quality surveillance of the continuous yarn (1) through evaluation of the yarn tension is effected when the yarn tension has reached a preselected setpoint range and preferably when subsequently a preselected control testing time Z3 has elapsed.

6. Method according to claim 5,
characterized in that
the evaluation signal is the current relative standard deviation (variation coefficient (cv)) which is formed from the current measured value of the yarn tension and the mean value continuously formed therefrom.

7. Method according to claim 5,
characterized in that
the evaluation signal is the standard deviation S which is formed from the root of the squared difference between the current measured value of the yarn tension and its mean value.

8. Method according to one of claims 1 to 8,
characterized in that
the uncontrolled friction force is preselected by a manipulated variable from the control loop of the yarn tension.

9. Method according to claim 9,
characterized in that
the manipulated variable is in each case differently preselectable in dependence upon the relevant exceptional operating situation.

10. Method according to claim 10,
characterized in that
the manipulated variable in the event of a power cut is selectable differently to the manipulated variable in the case of yarn piecing.

11. Method according to one of claims 9 to 11,
characterized in that
a manipulated variable is preselected which substantially determines the friction force components acting upon the yarn (1).

12. Method according to one of claims 9 to 11,
characterized in that
a manipulated variable is preselected which substantially determines the contact pressure of the friction false-twisting device (6).

13. Method according to one of claims 9 to 13,
characterized in that
a speed-dependent manipulated variable is preselected, whose specified value lies below the setpoint value in the case of operating speed.

14. Method according to claims 9 to 14,
characterized in that
in the event of a power cut, the control loop receives a backup supply from an emergency power network, and that
a predetermined parameter value in the vicinity of the working point is preselected as an uncontrolled manipulated variable.

15. Method according to claim 15,
characterized in that
the parameter value of the manipulated variable is continuously determined and stored during normal operation, and that
in the event of a power cut, the last stored parameter value represents the uncontrolled manipulated variable.

## Revendications

1. Procédé pour le réglage de la force de traction du fil en marche (1) dans une machine de texturation par fausse torsion, comprenant dans l'ordre
- une bobine de livraison (2) pour la levée du fil (1),
- un premier délivreur (3),
- une plaque de chauffage (4),
- une plaque de refroidissement (5),
- un dispositif fausse torsion par friction (6),
- un organe de réglage de la force de friction (7) et
- un dynamomètre de la traction du fil (8), dont le signal initial pendant la marche normale permet de régler la force de friction du dispositif fausse torsion par friction (6) au moyen de l'organe de réglage de la force de friction (7),
caractérisé en ce que
le réglage de la force de traction du fil en dehors de la marche normale, notamment au ralentissement de la machine à la suite d'une coupure de courant et/ou à l'occasion de l'étalage du fil, est désactivé et une force de friction non-réglée est donnée.

2. Procédé selon la revendication 1,
caractérisé en ce que
la force de friction est réglée sur une valeur plus grande que zéro, mais plus basse qu'est la valeur pendant la marche normale, pour être augmentée après, de préférence en continu ou par degrés.

3. Procédé selon la revendication 1,
caractérisé en ce que,
pour l'étalage du fil, le réglage de la force de traction est mis en action après une durée de démarrage toujours pareille et prédéfinie.

4. Procédé selon la revendication 1,
caractérisé en ce que
le réglage de la force de traction du fil est activé en fonction du mesurage de la force de traction du fil et d'un signal déduit, lorsque la force de traction du fil a atteint le domaine d'une valeur prescrite.

5. Procédé selon l'une quelconque des revendications 3 à 5,
caractérisé en ce que
la surveillance de la qualité du fil en marche (1) qui est pratiquée au moyen de l'évaluation de la force de traction du fil, commence au moment où la force de traction a atteint le domaine prédéfini d'une valeur prescrite et, de préférence, au moment où un temps de test prédéfini Z3 s'est écoulé.

6. Procédé selon la revendication 5,
caractérisé en ce que
le signal est l'écart type relatif et continu ( coefficient de variation (cv) ) qui est constitué par la valeur mesurée continue de la force de traction du fil et par la valeur moyenne y déduite en continu.

7. Procédé selon la revendication 5,
caractérisé en ce que
le signal est l'écart type S résultant de la racine de la différence élevée au carré entre la valeur mesurée continue de la force de traction du fil et sa valeur moyenne.

8. Procédé selon l'une quelconque des revendications 1 à 7,
caractérisé en ce que
la force de friction non-réglée est prédéfinie par une sortie réponse du circuit de réglage de la force de traction du fil.

9. Procédé selon la revendication 8,
caractérisé en ce que
la sortie réponse peut être choisie de façon différente selon les circonstances, en fonction d'une marche exeptionnelle quelconque.

10. Procédé selon la revendication 9,
caractérisé en ce que
la sortie réponse peut être choisie de façon différente au cas d'une coupure de courant ou au cas de l'étalage du fil.

11. Procédé selon l'une quelconque des revendications 8 à 10,
caractérisé en ce
qu'on choisit une sortie réponse qui détermine notamment les composantes de la force de friction agissant sur le fil (1).

12. Procédé selon l'une quelconque des revendications 8 à 10,
caractérisé en ce
qu'on choisit une sortie réponse qui détermine notamment la force de pression du dispositif fausse torsion par friction (6).

13. Procédé selon l'une quelconque des revendications 8 à 12,
caractérisé en ce
qu'on choisit une sortie réponse dépendant de la vitesse de rotation dont la valeur prévue est au-dessous de la valeur prescrite pour la vitesse de rotation pendant la marche normale.

14. Procédé selon l'une quelconque des revendications 8 à 13,
caractérisé en ce que
- le circuit de réglage est égalisé - au cas d'une coupure de courant - par un groupe électrogène de secours et
- qu'on choisit, comme sortie réponse non-réglée, une valeur-paramètre prévue à proximité du point de fonctionnement.

15. Procédé selon la revendication 14,
caractérisé en ce que
- la valeur-paramètre de la sortie réponse est prise et mémorisée en continu pendant la marche normale et que
- au cas d'une coupure de courant la valeur-paramètre dernièrement mémorisée représente la sortie réponse non-réglée.
